# EUROPEAN PATENT APPLICATION

(11) **EP 3 144 598 A1**
(43) Date of publication of application: **22.03.2017**
(21) Application number: 16187993.7
(22) Date of filing: 09.09.2016
(51) Int. Cl.: F24F 1/00, A61L 9/00, B05B 17/00, F24F 3/16, F24F 7/007

(54) **CLEANING SYSTEM AND METHOD FOR CONTROLLING INDOOR HAZARDOUS SUBSTANCES**

(30) Priority: 15.09.2015 CN 201510587512
(71) Applicant: Tai, Chi Pang, Kowloon (HK)
(72) Inventor: TAI, Chi Pang, Kowloon (HK); KWONG, Kam Yu, Kowloon (HK)
(74) Representative: Marks & Clerk (Luxembourg)

(57) **Abstract**

A cleaning system for controlling indoor harmful substances comprising a housing containing a controlling device to operate the cleaning system; an atomization device atomizing the cleaning liquid; and an air circulation device generating vortex flow to carry the atomized cleaning liquid for indoor circulation, wherein the counterforce of the vortex flow is sent to the inlet of the air circulation device. The cleaning system for controlling harmful substance in the present invention provides multiple advantages which can effectively incorporate large-areal disinfection, sterilization and sterilization by comprehensively transferring the atomized leaning liquid to every corner of the space through the air circulation device and improve the cleaning effectiveness. Meanwhile, the cleaning system in the present invention can be arranged with a remote controlling device so that users can focus on cleaning the local infected area without directly contacting the infected areas so as to reduce the risk of exposing users to the infected space.

## Description

### Field of Invention

The present invention relates to a cleaning system and method for controlling indoor harmful substances. More specifically, it relates to a cleaning system and method for dispersing atomized cleaning liquid in indoor environments.

### Background of Invention

In order to remove indoor pathogenicity substances such as bacterial, virus and fungus, people commonly use ultraviolet ray for indoor space disinfection and sterilization. The ultraviolet ray is commonly used in operating room, dispensary room and the likes in hospitals. However, as the ultraviolet ray could not cover hidden areas completely, extra operations are needed to clear up the site first in order to sufficiently expose the surface to be disposed under the ultraviolet ray during the disinfection. Furthermore, this method cannot be applied to infected sites where no ultraviolet lights are installed. For highly dangerous sites which are suspected to be infected, workers are usually deployed who wear heavy protective clothes and hand-hold cleaning tools to spray cleaning liquid indoor. This requires devoting a great amount of workers and disinfectors for disinfection and sterilization, and the workers also need to be trained so as to ensure that they are familiar with the operations to ensure their own safety.

Therefore, there is still required a highly effective system and method for controlling indoor harmful substances.

### Summary of Invention

Therefore, the present invention provides a cleaning system for controlling indoor harmful substances which overcomes the above-mentioned technical problem.

In one aspect, the present invention provides a cleaning system for controlling indoor harmful substances, which includes a housing containing a controlling device to operate the cleaning system; an atomization device atomizing the cleaning liquid; and an air circulation device generating a vortex flow to carry the atomized cleaning liquid for indoor circulation, wherein a counterforce of the vortex flow sends air to the inlet of the air circulation device.

Preferably, the air circulation device is located outside of the housing and the atomization device is at least partly arranged inside of the housing. The atomized gas outlet of the atomization device is adjacent to the air circulation device.

In one implementation, the air circulation device contains an air circulation fan. Preferably, the air inlet of the air circulation fan is configured with an air exhaust accelerator to improve the air exhaust speed. Optionally, the air circulation fan includes a flabellum and a fan cover. The fan cover is configured to offset the air flow generated by the flabellum so as to form the vortex flow. The flabellum has a curvature which facilitates formation of the vortex flow.

Preferably, the air circulation fan is connected to the housing via a support. The air circulation fan rotates around the support.

In one implementation, the atomization device includes a fluid reservoir for storing the cleaning liquid and an atomizer for atomizing the cleaning liquid. The fluid reservoir is fluidly connected to the atomizer. Optionally, the fluid reservoir is fluidly connected to the atomizer via an infusion tube and the fluid reservoir is formed with one or more cavities.

Preferably, the atomizer is an ultrasonic atomizer and the cleaning liquid is chosen from at least one of antibacterial agent, antiviral agent, antifungal agent and pesticide.

Furthermore, the controlling device contains a micro-processor connected to a circuit board. The controlling device further includes a signal transmitter and a signal receiver which are configured to communicate wirelessly with a remote controller. Preferably, the controlling device further contains at least one of timer, a sensor, and a videographer.

Preferably, the cleaning system further includes a shifting device to change the operating position of the cleaning system. Preferably, the shifting device contains wheels and a handle.

Accordingly to another aspect of the present invention, there is provided a method for controlling indoor harmful substances, which includes: an arrangement step which arranges the cleaning system in the indoor space, wherein the cleaning system includes an atomization device and an air circulation device; an atomization step which atomizes the cleaning liquid via the atomization device; and an air circulation step which generates a vortex flow via the air circulation device so as to carry the atomized cleaning liquid for indoor circulation.

Preferably, the atomized gas outlet of the atomization device is adjacent to the air circulation device.

In one embodiment, the air circulation device contains an air circulation fan. Preferably, the air inlet of the air circulation fan is configured with an air exhaust accelerator to improve the air exhaust speed. Preferably, the air circulation fan includes a flabellum and a fan cover. The fan cover is configured to offset the air flow generated by the flabellum so as to form the vortex flow. The air circulation fan contains a pivot and can rotate around the pivot. The flabellum contains a curvature which facilitates formation of the vortex flow.

In another aspect, the atomization device contains a fluid reservoir for storing the cleaning liquid and an atomizer for atomizing the cleaning liquid. The fluid reservoir is fluidly connected to the atomizer. Preferably, the cleaning liquid is chosen from at least one of antibacterial agent, antiviral agent, antifungal agent and pesticide.

Besides, the cleaning system contains a controlling device.

In one embodiment, the controlling device is connected to the atomization device and the air circulation device. Preferably, the method further contains a step of wirelessly communicating by using a remote controller and the controlling device of the cleaning system.

Therefore, the cleaning system for controlling harmful substances according to the present invention provides a number of advantages, one of which is to effectively achieve large-area disinfection, sterilization and sterilization by comprehensively transferring the atomized cleaning liquid to every corner of the space through the air circulation device, and thus improving the cleaning effectiveness. Meanwhile, the cleaning system in the present invention can be arranged with a remote controlling device so that users can target the cleaning to locally infected area without directly contacting the infected areas, so as to reduce the risk of exposing users to the infected space. Besides, users can configure the system with a daily timing and positioning cleaning procedure by setting the cleaning time according to requirements to ensure the cleanness of the operation area and thereby avoid problems caused by ignorance of cleaning.

The present invention is especially helpful to process infected indoor space. It can decrease the level of the harmful substances to a low level of risk or even harmlessness in a large area by positioning the system in the infected space to facilitate workers to collect and analyze and so on. Besides, with regard to different infection situations such as different harmful sources (bacterial, virus, harmful fungus and insect and so on), users can arrange different kinds of cleaning liquid (including the disinfector, sterilant, pesticide and antifungal agent and so on) for the system to conduct more well-directed cleaning works.

### Brief Description of Accompanying Drawings

The effects and advantages of the present invention can be further understood by referring to the remained part and figures of the present specification and the marker of the same component in the figures. Under some circumstances, sub-markers are placed following a certain marker and end dash to present one of many similar components. In the present text, similar or same components in different embodiments (such as 100, 200) are matched with markers with a difference of 100 to clearly show the similarity.
Figure 1 illustrates the schematic diagram of the cleaning system of controlling indoor harmful substance in one embodiment in the present invention.
Figure 2 illustrates the side view of the cleaning system shown in Figure 1, wherein part of the housing is removed.
Figure 3A illustrates the arrangement schematic diagram of the cleaning system shown in Figure 1, wherein the gas outlet of the air circulation fan is arranged to be parallel to the atomized gas outlet to face the same direction therewith.
Figure 3B illustrates the arrangement schematic diagram of the cleaning system shown in Figure 1, wherein the gas outlet of the air circulation fan is arranged to be inclined to the housing top.
Figure 3C illustrates the arrangement schematic diagram of the cleaning system shown in Figure 1, wherein the gas outlet of the air circulation fan is arranged to be at right angle with respect to the atomized gas outlet and the housing top, so as to allow the flow to upward exhaust in the housing top;
Figure 4 illustrates the schematic diagram of the cleaning system of controlling indoor harmful substances according to another embodiment in the present invention;
Figure 5 illustrates the side view of the cleaning system shown by Figure 4;
Figure 6 illustrates the embodiment of the controlling device in the cleaning system shown by Figure 4; and
Figure 7 illustrates the pathway of the generated vortex flow when the cleaning system in the present invention operates.

### Detailed Descriptions of Preferred Embodiments

Embodiments in the present invention adopt the combination of an atomization device and an air circulation device to comprehensively transport the atomized cleaning liquid to every corner of the space. The distinguishing advantages and merits provided by each embodiment can be easily known from the descriptions below.

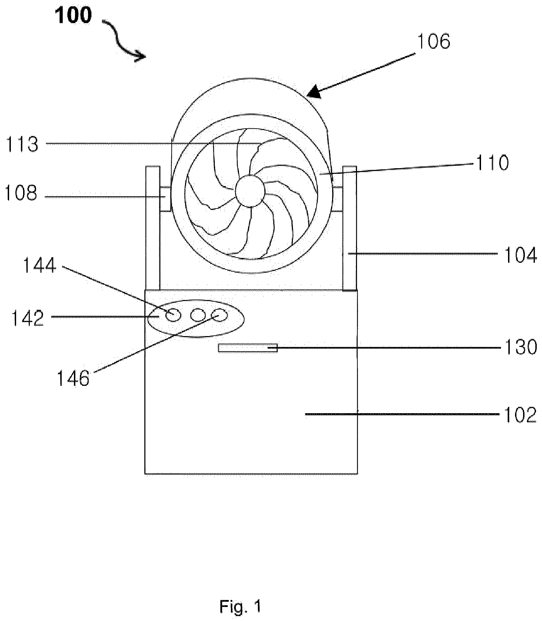

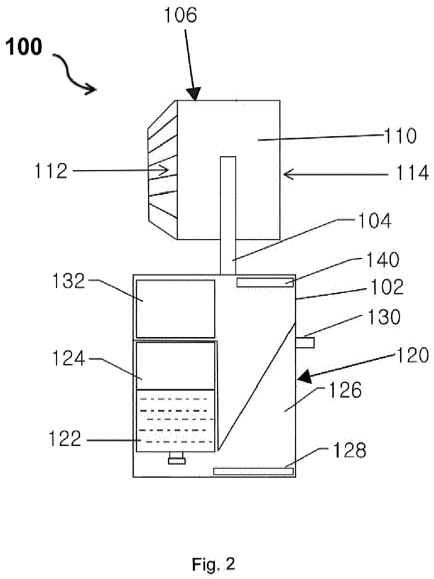

By referring to Figure 1 and 2, the first embodiment of the present invention provides a cleaning system 100 for controlling indoor harmful substances. The cleaning system 100 contains a housing 102 which is arranged with a support 104 to support the air circulation device 106. In the present embodiment, the air circulation device 106 is an air circulation fan 110 which can be pivotally connected to the pivot 108 on the support 104 to facilitate angle adjustment of the air circulation fan 110. The air circulation fan 110 contains a flabellum 113 and a fan cover 112 to generate the vortex flow, and is arranged with an air suction accelerator (not shown) in the gas inlet to accelerate the exhausting speed. Specifically, the flabellum is wide and possesses a streamline curvature capable of facilitating the formation of the vortex flow, so as to enlarge the contacting area with the suctioned air. Besides, the fan cover 112 is configured to offset the vortex flow generated by the flow generated by the flabellum 113.

As shown in Figure 2, inside of the housing 102 is further configured an atomization device 120 which contains a liquid reservoir 124 for storing cleaning liquid 122 and is fluidly connected to the adjacent atomization cavity 126. The bottom of the atomization cavity 126 is configured with an atomizer 128 to atomize the cleaning liquid 122. In the present embodiment, the atomizer 128 is an ultrasonic atomizer to atomize the liquid with ultrasonic sound. One side wall of the atomization cavity 126 close to the housing 102 is arranged with an atomized gas outlet 130 which is connected to the outside environment. Besides, the atomized gas outlet 130 is close to the air circulation fan 110 to facilitate the vortex flow generated by the air circulation fan 110 carrying the atomized cleaning liquid.

Besides, inside of the housing 102 is configured with a controlling device 140 connected to the inside power source 132, which contains a microprocessor connected to the circuit board. The circuit board is electrically connected to the atomizer 128 inside of the housing 102 and the air circulation fan outside of het housing 102, so as to control the operation of the atomizer 128 and the air circulation fan. Meanwhile, the controlling device 140 is connected to the controlling panel 142 outside of the housing 102. The controlling panel 142, as shown in Figure 1, contains a button 144 and an indicator light 146 to operate the cleaning system and reflect the state of the cleaning system such as active, pausing and shutdown.

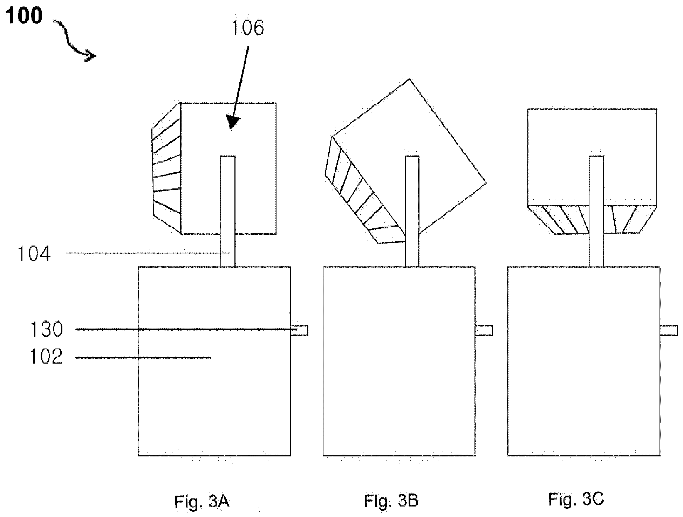

As shown in Figure 3A to 3C, the air circulation fan of the cleaning system 100 can rotate up and down relative to the pivot 108 of the support 104 so as to generate vortex flow from different angles. The rotation can be adjusted manually by users, or by communicating with the controlling device 140, to adjust the angle of the air circulation fan relative to the pivot 108 of the support.

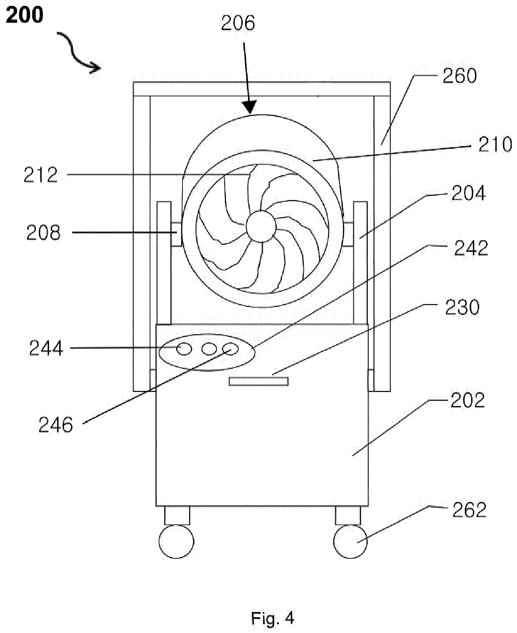

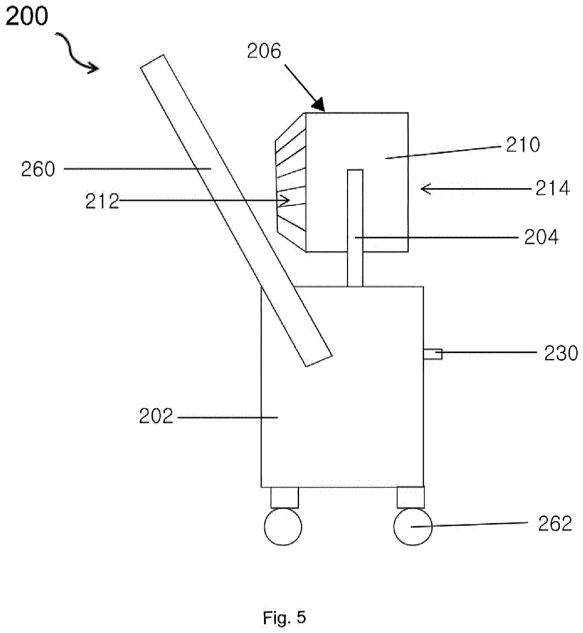

Figures 4 and 5 show another embodiment in the present invention which provides a cleaning system 200. The cleaning system 200 is substantively similar with the former-mentioned cleaning system 100 and the difference lies in that the cleaning system 200 is arranged with a moving device. The cleaning system 200 is connected with a handle 260 in both sides of the housing 202 and the bottom of the housing 202 is mounted with a wheel 262 allowing users either manually drive the cleaning system 200 to an desired position, or modify the operating position of the cleaning position during the cleaning work procedure. Besides, the wheel 262 of the cleaning system 200 is electrically connected to the controlling device 240 through a driving device including a motor and so on. The controlling device further includes a signal transmitter and a signal receiver that are connected to the microprocessor and are wirelessly connected to the remote controller. Therefore, users can control the driving device through a remote controller so as to move the wheel 262 change the operating position of the cleaning system 200.

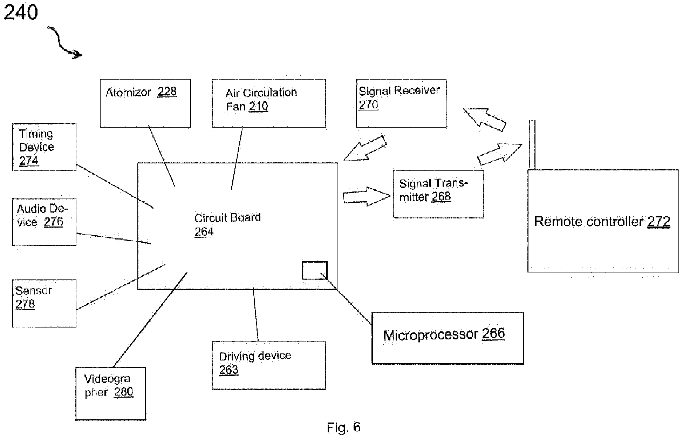

Figure 6 shows one embodiment of the controlling device 240 used for the cleaning system 200. The circuit board 264 of the controlling device is connected to the microprocessor 266, and simultaneously connected to the atomizer 228, the air circulation fan 210 and the driving device 263 through electronic connection to control the cleaning system 200 via the microprocessor 266. Besides, the microprocessor 266 is connected to the signal transmitter 268 and the signal receiver 270 to communicate wirelessly to the outside, which can be exemplified by that the operation parameter of the cleaning system 200 is sent to the remote controller 272 of users to further allow users to remotely control the operation of the cleaning system 200. Besides, as shown in Figure 6, the controlling device 240 is further connected to a timing device 274 (such as a timer and so on), a audio device 276, a sensor 278 and a videographer 280 to record the information for the operating procedure and senses variable parameters such as temperature and the harmful substances level in the operating environment and sends reminder signal to users and so on.

When implementing the embodiments 100 and 200 of the above-mentioned cleaning system in the present invention, users can arrange the cleaning system in to the indoor space to be disposed. Users initiate the cleaning system through the operation panel arranged on the housing or a remote controller. The controlling device initiates the atomizer according to instructions to atomize cleaning liquid sent into the atomization cavity as micro-particles which overflows the atomization cavity and is exhausted through the atomized gas outlet to the outside of the housing. Meanwhile, the controlling device initiates the air circulation device whose flabellum rotates in a high speed and improves the air suction speed by cooperating with an air suction accelerator configured at the gas inlet.

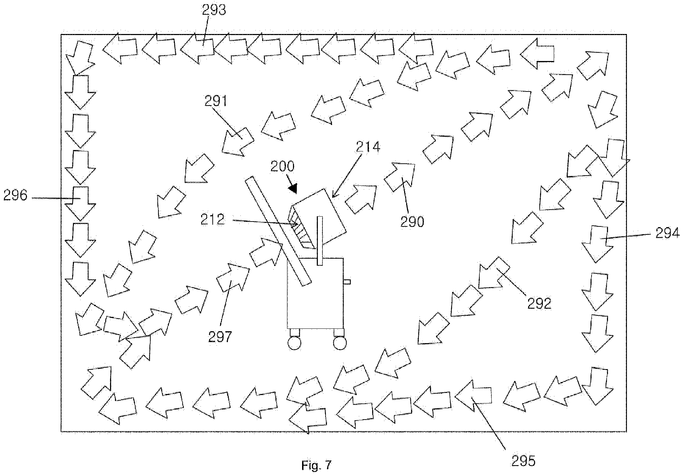

As shown in Figure 7, the vortex flow 290 is spread to the entire indoor space by counterforce. The vortex flow 290 is generated in the gas outlet 214 of the air circulation fan 210 and is sent to the indoor circumstance. When the flow 290 contacts with an object such as when striking the wall, the generated vortex counterforce divides the flow 290 into multiple branch flows 291, 292, 293 and 294 which are spread into the space in all direction. Although the flow 290 is divided into multiple branch flow, as the original vortex flow 290 possesses a huge kinetic energy, the branch flows outwardly spread at the striking points of the vortex flow in the form of small sized airstreams. Then multiple small sized airstreams contact with other indoor objects and continues to subdivide and spread 295 and 296, which is followed by converging at the gas inlet 212 close to the air circulation fan. The air suction accelerator at the as inlet 212 of the air circulation fan also contributes to the flow convergence 297 and exhaust the air to generate the vortex flow 290.

Accordingly, when the atomized gas is sent to the indoor space through the atomized gas outlet, the air circulation fan close to the atomized gas outlet can effectively and fast overflow the air loaded with micro-particles in the indoor space, and reflux the air to the gas inlet by counterforce. Thereby, air carrying the micro-particles of the cleaning liquid is allowed to circulate in the indoor circumstance and reduce or eliminate harmful substances to living bodies.

When adopting the cleaning system arranged with a moving device, users can move the cleaning system to other operating circumstances for cleaning procedures. Users can manually drive the cleaning system or operate the moving device with the remote controller. When using the remote controller, users can also trace and monitor the position of the cleaning system and adjust the operation parameters in real-time through the display of the remote controller. For example, the videographer or vidicon installed in the cleaning system records, monitors, and adjusts the angle of the air circulation fan, and parameters of the atomizer, and selects the atomized cleaning liquid and so on. Furthermore, the level of harmful substances measured by the sensor can be returned to users in real-time. Therefore, the cleaning system in the present invention also provides conveniences in multiple aspects when ensuring health and and safety for the operating workers.

Besides, as shown in embodiments in Figure 6, the controlling device is connected to the audio device which contains a phonetic recognition program. Users can orally command the cleaning system so that audio device captures the command, analyzes through the phonetic recognition program, and sends the analyzed data to the micro-processor, which operates corresponding to the command through electric circuits.

It can be understood that the present cleaning system can be arranged in operating environments by other methods such as mounting it on the wall and fixing it on other indoor devices and so on. Under some circumstances, when a large scale of infectious diseases outbreak, a movable cleaning system can be adopted and it can be configured with the moving device. Users only needs to guide the movable cleaning system by using the remote controller and enters the infectious area for cleaning so as to relieve human resource load and reduce the safty risk while improve the cleaning efficiency. Similarly, areas liable to be infected such as dispensary rooms and operation rooms in the wards can be mounted with a fixed cleaning system, such as wall-hung type of cleaning system. The cleaning process can be configured in advance through the controlling device such as by pre-determining the initiating time, cleaning time and cleaning liquid, as a result the daily timing and positioning cleaning procedures can thereby be conducted.

Preferably, but not limited to, the air circulation device can be configured on top of the housing and at a position close to the atomized gas outlet. The atomizer can be chosen from any known atomizers to those skilled in the art such as an ultrasonic one, gas impression one and so on. Besides, the atomizer is preferably configured at a position close to the atomized gas outlet to facilitate the exhaust of the already atomized liquid particles and further avoid the generation of condensation because of remaining too long in the housing. In some embodiment, the adopted atomizer can be replaceable. Therefore, when the atomizer is damaged or fails occurring from long time use, users can simply replace the atomizer or the atomizer components so that there is no need to replace the entire cleaning system.

In other embodiments in the present invention, the liquid reservoir in the atomizer device can be configured with one or more cavities to contain one or more types of cleaning liquid. Besides, the volume of the liquid reservoir can be mutually different. Furthermore, the liquid reservoir and the atomizer containing the cleaning liquid can be fluidly connected by one or more infusion tube and the infusion tube transports the cleaning liquid to the atomization cavity so as to atomize the cleaning liquid through the atomizer. Optionally, the atomizer can also include one or more atomized gas outlet. It can be understood that other conventional components such as drain outlet and so on can be arranged in the atomization device to provide convenience for the operation of the atomization device. Examples include that the drain outlet is configured at the bottom of the atomization cavity so as to discharge redundant liquid and so on. Those skilled in the art can know that each composition can be used without departing from the nature of the present invention.

The cleaning liquid can be any liquid capable of reducing or eliminating the harmful substances to the living body, wherein it may include anti-virus agent, anti-bacterial agent, sterilization agent and pesticide. The anti-infectors refer to substances that exert anti-inference and inhibiting function on the specific harmful virus. The anti-bacterial agent includes anti-bacteria agent and disinfectors and so on, which will kill harmful bacteria and bacteria spore and so on in the circumstance or decrease the level to one that is not harmful to the human body. Anti-bacteria agent refers to substances of killing or inhibiting and/or fungal spore. The pesticide contains the insecticide, the miticide and herbicide and so on, which can be any substances of preventing, driving out, inhibiting or controlling insects, rodents or weeds and so on harmful to the circumstance or ones that regulates the plant growth.

Specifically, the anti-virus agent, anti-bacteria agent and sterilization agent can be chosen from silver dihydrogen citrate generic ones, bleaching water generic ones, quaternary ammonium generic ones, alcohol generic ones, aldehyde generic ones, hydrogen peroxide generic ones, phenol generic ones, biguanides generic ones, plant source generic ones, echinocandins generic ones, polyenoid generic ones, triazole generic ones, alkyl amine generic ones and methyl thiazolyl tetrazolium generic ones and so on. The pesticides mainly include two types the inorganic matters and organic pesticide, wherein inorganic matters include copper agents, sulphur agents, silver agents and arsenical agents and so on; organic pesticides include pyrethrin, synthesized pyrethrin, microorganism pesticide, plant pesticide, organic sulphur agent, carbamic acid ester, organic-chlorine and organic-phosphorous agent and so on. Among one of preferable embodiments in the present invention, the adopted cleaning liquid can be PureGreen 24 disinfector containing silver dihydrogen citrate which can still remain the ability of disinfecting, sterilization and antifungal after the ultrasonic atomizer functions.

In other embodiments in the present invention, the cleaning system can contain one or more sensors to sense different parameters of indoor circumstances such as temperature, humidity, the level of harmful substances the concentration of the cleaning liquid micro-particles in the indoor circumstance. Specifically, the sensor sensing the concentration of the cleaning liquid micro-particles in the indoor circumstance can be installed outside of the housing and far from the atomized gas outlet. Therefore, the air circulation efficiency and the atomization efficiency of the cleaning system can be analyzed by the measured data and the atomization device or air circulation device can adjusted according to data. For example, if the measured concentration is lower than the predicted level, users can adjust the atomizer to accelerate the atomization speed by controlling the device; if the measured concentration reaches the predicted level, it can postpone the atomization speed temporarily. Furthermore, after the controlling system analyzing the measured data, the corresponding measurements can be conducted by itself, such as automatically adjusting the atomization device and/or air circulation device and/or sending reminding signals to users.

Besides, those skilled in the art shall know clearly that the controlling device comprises the preferably configured waterproof and/or antifogging designs such as the circuit board and circuit in order to prolong the service life of the controlling device in the present invention. Furthermore, the housing and device and components of the cleaning system can be chosen from anti-acid-base, anticaustic materials in order to prolong the service life the system. Similarily, the housing, device and components can be coated with a protective layer to provide the anticaustic function. Thereby, the cleaning system can be resistant to substances that strongly anti-corrosive such as acid, base and salt, which can facilitate the cleaning system with the corrosive cleaning liquid such as strong acid and strong base to prolong the service life of the system.

As for the power source, those skilled in the art can appreciate that the power source connected to the controlling device can be internal storing electricity power source or external power supply.

Therefore, after introducing several embodiments, those skilled in the art can appreciate that different modifications, other structures and equivalents can all be used without departing the nature of the present invention. Correspondingly, the above-mentioned descriptions shall not be regarded as limitations to the scopes of the present invention ascertained by the claims in the present application.

## Claims

1. A cleaning system for controlling indoor harmful substances, comprising:
a housing which accommodates a controlling device to operate the cleaning system;
an atomization device for atomizing the cleaning liquid; and
an air circulation device generating a vortex flow to carry the atomized cleaning liquid for indoor circulation, wherein a counterforce of the vortex flow send air to an inlet of the air circulation device.

2. The cleaning system according to Claim 1, **characterized in that** that the air circulation device is located outside of the housing and the atomization device is at least partially arranged inside the housing; optionally, the atomized gas outlet of the atomization device being adjacent to the air circulation device.

3. The cleaning system according to Claim 2, **characterized in that** the air circulation device comprises an air circulation fan; preferably, the air inlet of the air circulation fan configured with an air suction accelerator to improve the air suctioning speed; and additionally or alternatively, the air circulation fan comprising a flabellum and a fan cover, the fan cover configured to offset the air flow generated by the flabellum so as to form the vortex flow.

4. The cleaning system according to Claim 3, **characterized in that** the air circulation fan is connected to the housing via a support; preferably, the air circulation fan rotating around the pivot of the support.

5. The cleaning system according to Claim 3 or Claim 4, **characterized in that** the flabellum comprises a curvature which facilitates the formation of the vortex flow.

6. The cleaning system according to anyone of the preceding claims, **characterized in that** the atomization device comprises a fluid reservoir for storing the cleaning liquid, and an atomizer for atomizing the cleaning liquid; wherein the fluid reservoir is fluidly connected to the atomizer; preferably, the fluid reservoir is fluidly connected to the atomizer via an infusion tube; additionally or alternatively, the fluid reservoir arranged with one or more cavities.

7. The cleaning system according to Claim 6, **characterized in that** the atomizer is a ultrasonic atomizer.

8. The cleaning system according to any one of the preceding claims, **characterized in that** the cleaning liquid is chosen from at least one of antibacterial agent, antiviral agent, antifungal agent and pesticide.

9. The cleaning system according to any one of the preceding claims, **characterized in that** the controlling device comprises a microprocessor connected to a circuit board.

10. The cleaning system according to Claim 9, **characterized in that** the controlling device further comprises a signal transmitter and a signal receiver which are used to communicate wirelessly with a remote controller.

11. The cleaning system according to Claim 9 or Claim 10, **characterized in that** the controlling device further comprises at least one of a timer, a sensor, and a videographer.

12. The cleaning system according to any one of the preceding claims, further comprises a moving device to change the operating position of the cleaning system.

13. The cleaning system according to Claim 12, **characterized in that** the moving device comprises wheels and a handle.

14. The cleaning system according to Claim 12 or Claim 13, **characterized in that** the moving device is connected to the controlling device so that users can remotely control the shifting device.

15. A method for controlling indoor harmful substances comprising:
an arrangement step which arranges the cleaning system in the indoor space, wherein the cleaning system comprises an atomization device and an air circulation device;
an atomization step which atomizes the cleaning liquid by the atomization device; and
an air circulation step which generates vortex flow via the air circulation device so as to carry the atomized cleaning liquid for indoor circulation.
